# EUROPEAN PATENT APPLICATION

(11) **EP 4 574 112 A1**
(43) Date of publication of application: **25.06.2025**
(21) Application number: 24220114.3
(22) Date of filing: 16.12.2024
(51) Int. Cl.: A61F 9/008

(54) **OPHTHALMOLOGICAL SYSTEM FOR ABLATIVE REMOVAL OF EYE COLORANT ARRANGED IN THE CORNEA OF AN EYE OF A PATIENT AND COMPUTER PROGRAM PRODUCT FOR CONTROLLING A PROCESSOR OF THE OPHTHALMOLOGICAL SYSTEM FOR ABLATIVE REMOVAL OF EYE COLORANT**

(30) Priority: 21.12.2023 CH 14562023
(71) Applicant: Ziemer Ophthalmic Systems AG, 2562 Port (CH)
(72) Inventor: RATHJEN, Christian, 28197 Bremen (DE); STEINLECHNER, Michael, 8006 Zürich (CH)
(74) Representative: Rentsch Partner AG

(57) **Abstract**

The present disclosure relates to an ophthalmological system (100) for ablative removal of eye colorant (203) arranged in a cornea (202) of an eye (201) of a patient (200), the ophthalmological system (100). The ophthalmological system (100) comprises a position measurement system (101) configured to determine an area (205) in the cornea (202) comprising the eye colorant (202), a laser source (102) configured to generate a pulsed laser beam (T), a focusing optics (103) configured to make the pulsed laser beam (T) converge onto a focal spot (S) in the cornea (202), a scanner system (104) configured to deflect the pulsed laser beam (T), and an electronic circuit (105) configured to control the scanner system (104) to direct the focal spot (S) to process the determined area (205) comprising the eye colorant (202) in the cornea (202) for ablative removal of the eye colorant (204).

## Description

### FIELD OF THE DISCLOSRE

The present disclosure relates to an ophthalmological system for ablative removal of eye colorant arranged in a cornea of an eye of a patient and to a computer program product comprising a non-transitory computer-readable medium having stored thereon computer program core for controlling a processor of the ophthalmological system for ablative removal of eye colorant. The present disclosure relates in particular to an ophthalmological system comprising a laser source, focusing optics, a scanner system and an electronic circuit, which is configured to control the ophthalmological device.

### BACKGROUND OF THE DISCLOSURE

For the purposes of working on eye tissue by means of a laser beam, a work region is scanned by laser pulses by virtue of the pulsed laser beam being deflected in one or more scan directions by means of suitable scanner systems and converged onto a focal spot by a focusing optical module. In general, movable mirrors are used to deflect the light beams and/or the laser pulses, for example femtosecond laser pulses, said movable mirrors being pivotable about one or two scan axes, for example by way of galvano scanners, piezo scanners, polygon scanners, or resonance scanners. Further scan components, such as divergence modulators or z-modulators, are known for positioning and moving the focal spot in the eye tissue with respect to further scan axes.

US patent US 7,621,637 B2 describes exemplarily an apparatus for working on eye tissue, said apparatus having a base station with a laser source for producing laser pulses and a scanner, arranged in the base station, with movable deflection mirrors for deflecting the laser pulses in a scan direction. The deflected laser pulses are transferred via an optical relay system from the base station to an application head, the latter passing over a work region according to a scan pattern by means of a mechanically moved projection optical unit.

For processing or creating a pocket in the eye tissue, ophthalmological devices are known, such as scalpels or laser systems, which use scanner systems with multiple scan axes. The conventional available scanner systems enable to create a pocket within the cornea e.g. for removing a lenticule or for inserting a lens or for inserting cornea material.

Each person has an individual iris color distribution and iris color structure. The individual color and structure of the iris is determined by an individual composition of pigments across the iris of the eye. The eye color distribution of humans varies normally from dark brown to light blue. Different pigment concentrations on different areas of the iris may further determine the individual appearance of the iris. For example, a light blue center of the iris may be surrounded by a darker ring on the peripheral part of the iris.

Different diseases or defects of the iris may change the color of the iris or may affect the iris color distribution or the appearance of the iris. Further, it is possible that congenital diseases or defects affect the properties and geometrical extensions of the iris of the eye, such that the iris of the patient deviates from a normal ring shape. Such congenital diseases are for example albinism, coloboma or aniridia, further congenital diseases are also conceivable. In order to at least improve or affect the visual appearance of such an affected iris, it is possible to perform a so-called keratopigmentation. Keratopigmentation describes different kind of chirurgical methods to change the appearance of the iris by treating the cornea arranged in front of the respective iris of the eye. For example, it is possible to place a colored implant on the iris or to depigment the iris with a laser beam. Both chirurgical methods are dangerous for the iris and in particular, the second one is limited with respect to only brightening the already existing iris. Another possibility to amend the visual appearance of the iris is to place in the cornea of the respective eye, thus in front of the iris, ink, such that the ink covers partially or entirely the natural color of the iris. Over time, the ink or eye colorant within the cornea may bleach or fades out. In addition, the ink may slowly move into other regions or the cornea, which affect the visual appearance and could harm the eye of the patient. A removal or replacement of the eye colorant or ink might be necessary. Conventionally, the eye colorant or ink is removed manually by a medical professional, which opens the cornea for access to the respective region and which scrapes out the respective colorant material. This uncontrolled procedure is often additionally affects cornea tissue negatively which is healthy and should not be affected. Further, the procedure may harm the entire eye because the eye itself offers no boarders for the medical professional. In addition, the areas in the cornea comprising the eye colorant are conventionally determined by the medical professional, which is not very accurate, resulting in remaining eye colorant in the cornea after the removal procedure.

### SUMMARY OF THE DISCLOSURE

It is therefore an object of the present disclosure to provide an ophthalmological system for ablative removal of eye colorant arranged in a cornea, which addresses at least some of the disadvantages of the known prior art. In particular, it is an object of the present disclosure to provide an alternative and / or an improved ophthalmological system and a computer program product for controlling the ophthalmological system, which is in particular configured for ablative removal of eye colorant arranged in a cornea of a patient.

According to the present disclosure, these objects are addressed by the features of the independent claims. Moreover, further advantageous embodiments emerge from the dependent claims and the description.

According to the present disclosure, an ophthalmological system for ablative removal of eye colorant arranged in a cornea of an eye of a patient is specified. The ophthalmological system preferably comprises a position measurement system, a laser source, a focusing optics, a scanner system and an electronic circuit.

The position measurement system is configured to determine an area in the cornea comprising the eye colorant, preferably using image information of the cornea. In other words, the position measurement system is a system, which is configured to measure the eye of the patient, in particular to determine, which portions or areas of the eye tissue form which part of the eye, e.g. cornea, sclera, lens etc. In addition, the positon measurement system is configured to determine an area or a plurality of areas in the cornea comprising the eye colorant. The output of the position measurement system may be reference data describing the position of the eye colorant within the cornea and / or the position of the cornea within the respective eye.

The laser source is configured to generate a pulsed laser beam and the focusing optics is configured to make the pulsed laser beam converge onto a focal spot in the cornea and the scanner system is configured to deflect the pulsed laser beam to target locations in the cornea. The scanner system may comprise only one scanner assembly arranged in one housing, which provides the required functionalities. In another embodiment, the scanner system may comprise a plurality of scanner assemblies arranged in one or a plurality of housings, which provide in combination with each other the required functionality.

The ophthalmological system further comprises an electronic circuit configured to control the scanner system to direct the focal spot to process the determined area comprising the eye colorant in the cornea for ablative removal of the eye colorant arranged in the cornea. In other words, the electric circuit controls the scanner system to direct the focal spot such that the area of the cornea comprising the eye colorant is processed. Processing means that the laser energy is focused on the eye colorant, in particular on the area, comprising the eye colorant, such that the eye colorant is removed, in particular vaporized. The position measurement system is configured to determine the area comprising the eye colorant in a very precise manner. In particular, the position measurement system uses algorithms and / or input form medical professionals, for determining of the eye colorant comprising areas. E.g. images captured by the position measurement system are analyst by a respective software, in particular in combination with medical professionals for capturing all areas of the cornea, which comprise eye colorant. In addition, the software or the medical professional may limit the area to respective portions. In other words, the position measurement system advantageously provides accurate three-dimensional position information of eye colorant within the cornea of the eye, which is desired to be removed. The position measurement system may comprise a video capturing system (color camera), an optical coherence tomography (OCT) system, a scheimpflug device, and/or a structured light illumination system, which are configured to provide measurement data. Accordingly, the measurement data or positional reference data determined by the measurement system includes video data, including top view data (comprising two-dimensional images), and/or OCT data of the cornea (comprising three-dimensional tomography data). The measurement system may be configured to determine the positional reference data of the cornea and / or of the area, which comprises the eye colorant, in particular also in an applanated state of the cornea. The measurement system may be connected to and/or integrated with the electronic circuit, which is further configured to control ophthalmological device, in particular the scanner system, using the data. As mentioned, the position measurement system may be configured to determine by itself the area of the cornea comprising the eye colorant or the position measurement system may be used by a medical professional to determine the area comprising the eye colorant. The position measurement system may show a picture of the cornea to the medical professional, which determines solely or with the aid of the position measurement system software the area comprising the eye colorant. In another embodiment, the position measurement system may provide a preselection of an area comprising the eye colorant, which may be modified or adapted by the medical professional. The result e.g. the geometric position reference data, which determines the area comprising eye colorant, is further used by the ophthalmological system for the ablative removal of the eye colorant. The position measurement system may be a standalone system, which is not directly integrated in the ophthalmologic device, which comprises the laser source, scanner system etc. The standalone position measurement system may be a separate device, which forms part of the ophthalmological system, and which is used prior of the ablative removal. In another embodiment, the position measurement system may be integrated in the ophthalmologic device, which also comprises the laser source and the scanner system.

The scanner system is configured to control the movement of the focal spot very accurately such that only the area of the cornea, which comprises the eye colorant is processed or treated. The ophthalmological system according to the present disclosure offers therefore a safe and reliable treatment possibility for removing of eye colorant within the cornea, in particular compared to conventional systems and methods.

In an embodiment, the position measurement system is configured to determine position reference data of the cornea, position reference data of the area comprising the eye colorant to be treated and/or position reference data of the treated area, during processing or treating of the cornea, in particular continuously, such that treating, in particular the ablative removal of the eye colorant, is monitored, in particular continuously monitored. The position measurement system, in particular the OCT integrated in the respective ophthalmological device, might be configured to determine the position reference data of the cornea, position reference data of the eye colorant to be treated and position reference data of the treated area during processing or treating of the cornea. This is in particular performed continuously or at predetermined time intervals e.g. each millisecond. It is thereby possible that the treating, in particular the ablative removal of the eye colorant, can be monitored by the electronic circuit. It is further preferred that the monitoring is performed continuously or in real time e.g. each millisecond. The determined actual position reference data during treatment from the position measurement system may be continuously matched to expected data, in particular expected position reference data. In case the determined position reference data during treatment deviate from the expected data, in particular when the deviation reaches or fulfills a predetermined threshold, the electronic circuit might adjust the control data for the scanner system or the entire ophthalmological system, in particular in real time. In case a strong deviation e.g. surpassing the threshold by 25% or more is detected, the electronic circuit might be configured to interrupt the treatment.

In an embodiment, the laser source of the ophthalmological system used for ablative removal of the eye colorant comprises a solid-state laser source, excimer laser source and/or a femtosecond laser source. The laser source is in particular configured to provide the pulsed laser beam with the required laser power for the ablative removal of the eye colorant. Different eye colorants may require different laser parameters for an optimized removal. The laser source is preferably configurable such that the eye colorant is ablatively removable by the laser source.

In an embodiment, the electronic circuit is configured to control the scanner system to direct the focal spot to process the determined area such that only the eye colorant arranged in the cornea is removed. According to this embodiment, no cornea tissue is ablatively removed. In another embodiment, the electronic circuit is configured to control the scanner system to direct the focal spot to process the determined area such that the eye colorant and a limited area of surrounding cornea tissue is additionally removed. The limits are e.g. determined automatically or manually, preferably in dependence of the eye colorant properties and or the distribution of the eye colorant in the cornea. A widely and thin distributed eye colorant may require larger limits compared to a clearly concentrated spot of eye colorant, which is to be removed.

In an embodiment, the electronic circuit is configured to control a scanner device of the scanner system to move the focal sport with a scanning speed along a processing path generated based on the determined area comprising the eye colorant, thereby processing an eye colorant comprising surface within the cornea. The processing path determines the trajectory or the combination of trajectories, along which the focal spot of the pules laser beam is moved for processing the cornea tissue. The processing path is e.g. determined such that the desired area comprising the eye colorant is advantageously processed. The processing path may therefore comprise e.g. a plurality of parallel path segments or a plurality of circles. The processing path is e.g. determined by the electronic system such that the time for processing is optimized, e.g. reduced. The electronic circuit is preferably configured to automatically generate the processing path using the data from the position measurement system for an optimized processing of the area comprising the eye colorant.

In an embodiment, the processing path comprises a plurality of sub processing paths, which are generated based on the determined area comprising the eye or based on a plurality of areas comprising the eye colorant in the cornea. According to this embodiment, a plurality of sub processing paths form the processing path for one eye. It is e.g. conceivable that the cornea comprises two or more spots with eye colorant, which needs to be processed or removed. In this case, the electronic circuit is configured to determine a first sub processing path for processing the first spot and a second sub processing path for processing the second spot. At least during the movement from the first sub processing path to the second sub processing path the pulsed laser beam is switched off.

In an embodiment, the position measurement system is configured to determine a thickness and / or a depth position of the area comprising the eye colorant within the cornea, and wherein the electronic circuit is configured to move the focal spot to process the determined area using the determined thickness and / or depth position of the area comprising the eye colorant. The depth position is the position in z-direction within the cornea. As already mentioned, the eye colorant is not formed as a layer having an infinite small thickness. To the contrary, the eye colorant has a defined thickness and possibly also a varying thickness across the cornea. In order to affect as little cornea tissue as possible it is highly desirable to determine the thickness and depth position of the area comprising the eye colorant. This is in particularly advantageous determinable by the position measurement system comprising an OCT System. According to this embodiment, the area comprising the eye colorant is determined three-dimensional, which enables to advantageously ablatively remove the eye colorant. Thicker segments of the area are e.g. processed several times and thinner segments of the area are e.g. processed only once. The predetermined processing path is determined by the electronic circuit accordingly. Further, it is also conceivable that the laser power or other parameters of the laser are adjusted based on the determined thickness of the eye colorant, in particular such that the eye colorant may be advantageously entirely ablatively removed.

In a further embodiment, the position measurement system is configured to determine the thickness and / or a depth position of the area comprising the eye colorant within the cornea during processing or treating of the cornea, in particular continuously, such that treating, in particular the ablative removal of the eye colorant, is monitored, in particular continuously further using the thickness and / or depth position of the area comprising the eye colorant. It is thereby possible that the treating, in particular the ablative removal of the eye colorant, is monitored by the electronic circuit further using the thickness and or the depth position. The determined actual thickness or depth position data may be matched to expected data, in particular expected thickness or depth position reference data. In case the determined actual thickness or depth position data during treatment deviate from the expected data, in particular when the deviation reaches or fulfills a predetermined threshold, the electronic circuit might adjust the control data for the scanner system or the entire ophthalmological system, in particular in real time. Further, in case the thickness of the area comprising the eye colorant reaches zero or close to zero, the processing of the respective portion is stopped and may continue at another portion. Further, an additional buffer of cornea tissue in z-direction may additionally be treated such that the eye colorant is advantageously removed completely.

In an embodiment, the electronic circuit is further configured to control the scanner system to move the focal spot to form a cut extending at least partially from the outer surface of the cornea into the cornea, thereby forming a cornea flap, which is moveable for providing direct access to the area comprising the eye colorant in the cornea. In this embodiment, the area comprising the eye colorant is laid open or uncovered by forming the cornea flap. The cornea flap is the portion of the cornea, which at least partially covers the eye colorant when viewed along the z-direction of the eye towards the eye. It may be required that this portion of the cornea is unaffected by the ablative removal of the eye colorant. In order to achieve this and to ensure only the area comprising the eye colorant is processed, forming the cut and flapping the resulting cornea flap away could be used. The cut or the plurality of cuts, in particular the respective processing paths for forming the cornea flap is e.g. automatically determined by the electronic circuit using the position data of the area comprising the eye colorant from the position measurement system. It is to be noted that it is also possible to focus the pulsed laser beam through the cornea for ablative removal of the eye colorant without providing direct access. Forming the cornea flap and flipping the cornea flap away for direct access is optional. In case no cornea flap is formed, a venting access to the eye colorant may be formed enabling that the vaporized eye colorant may stream advantageously out of the cornea. The venting access may additionally be used for cleaning of the resulting cavity in the cornea.

The eye of the patient is a three dimensional object, which extends in three space direction, in particular in the x-, y- and the z-direction. The z direction is e.g. parallel with respect to the symmetry axis of the eye, parallel with respect to the visual axis of the eye or parallel with respect to a virtual axis extending crossing the center of the pupil and the apex of the cornea. Further, the z-direction might be perpendicular arranged to a plane of the eye, in particular the pupil plane of the eye. The x-y direction might be arranged such that a virtual plane, which is determined by the x and y direction is arranged coplanar or parallel with the iris or pupil plane. Other definitions might also be conceivable.

In an embodiment, the ophthalmological device, which is used for cutting the cornea flap corresponds to the ophthalmological device, which is used for the ablative removal of the eye colorant. In other words, a single ophthalmological device is used for cutting the cornea flap and for processing the area comprising the eye colorant by ablative removal.

The parameters of the pulsed laser beam are adapted respectively for an advantageous cutting and processing of the cornea of the eye.

In an embodiment, the ophthalmological system further comprises an access device configured to form the cut in the cornea, which extends from an outer surface of the cornea into the cornea thereby forming the cornea flap, which is moveable for providing direct access to the area comprising the eye colorant in the cornea. This embodiment differs from the previous described embodiment in that the cut in the cornea for forming the cornea flap is made by another device, namely the access device, and not by the device, which is used for the ablative removal. The access device is e.g. a surgical scalpel, a mikroceratom and/or a trepanic device.

In an embodiment, the access device is provided by a standalone access cutting ophthalmological device comprising a respective position measurement system, a respective laser source configured to generate a pulsed laser beam, a focusing optics configured to make the pulsed laser beam converge onto a focal spot in the cornea and a scanner system configured to deflect the pulsed laser beam to target locations in the cornea. This standalone access cutting ophthalmological device may additionally comprise an electronic circuit configured to control the scanner system to move the focal spot inside the cornea to form the cut extending at least partially from the outer surface of the cornea into the cornea, thereby forming the cornea flap, which is moveable for providing direct access to the area comprising the eye colorant. This standalone access cutting ophthalmological device may deviate from the ablative ophthalmological device in the laser source. The laser source of the access cutting ophthalmological device is selected for an advantageous cutting and the laser source of the ablative ophthalmological device is selected for an advantageous ablative removal. Both devices form part of the ophthalmological system. The position measurement system may be a standalone device or is integrated in the access cutting ophthalmological device. In case the position measurement system is a standalone device, the data from it may be provided to the access cutting ophthalmological device and the ablative ophthalmological device, in particular via a wired or wireless connection.

In an embodiment, the laser source of the access cutting ophthalmological device comprises a femtosecond laser source for producing femtosecond laser pulses, a solid-state laser source and/or an excimer laser source. In particular, the femtosecond laser source advantageously provides the desired laser properties for forming the cut in the cornea.

In an embodiment, the electronic circuit is configured to control the scanner system to move the focal spot inside the cornea to generate the cut such that a portion of the cornea flap remains in contact with the rest of the cornea, enabling that the cornea flap is flipable away for enabling access and flipable back for enabling to cover the area, which previously comprised the eye colorant. According to this embodiment, the cornea flap is not entirely separated from the rest of the cornea tissue. Preferably, at a peripheral point of the cornea is spared by the cut. This embodiment enables to advantageously position the cornea flap back after the ablative removal.

In an embodiment, the electronic circuit is configured to control the scanner system to move the focal spot inside the cornea to generate an at least partially annulus shaped cut, thereby forming an at least partially annulus shaped cornea flap, which is moveable for providing direct access to the eye colorant comprising location, and which keeps the center portion of the cornea unaffected. The eye colorant is most of the times arranged such that it covers the iris of the eye. The eye colorant has therefore the shape of a ring or annulus when viewed along the z-direction of the eye towards the eye. The center portion of the cornea, in particular the pupil portion, is normally free of eye colorant. This center portion is also the most important one regarding the visual sight of the eye. The light needs to travel as unaffected as possible through the center portion of the cornea for a good sight. According to this embodiment, the center portion is unaffected by the ablative removal and the forming of the cut for the flap. In other words, the center portion is spared during forming of the cornea flap.

In an embodiment, the electronic circuit is configured to control the scanner system to move the focal spot inside the cornea to generate the cut such the cut is arranged adjacent to the area comprising the eye colorant. The electronic circuit is in particular configured to control the scanner system such that the cut is immediately arranged adjacent to the area comprising the eye colorant. As mentioned, the eye colorant is distributed three-dimensional within the cornea, e.g. having thicker and thinner portions. In order to ablatively remove the eye colorant advantageously it is desired to place the cut also three-dimensional adjacently above the eye colorant. The cut has e.g. the shape of the top surface of the area comprising the eye colorant. In a further embodiment, the electronic circuit is configured to control the scanner system to move the focal spot inside the cornea to generate the cut such the cut is arranged with a predetermined offset to the area comprising the eye colorant. In this embodiment, the cut is formed at the offset, of e.g. 5-100 micrometers, away from the area comprising the eye colorant towards the anterior surface of the cornea. This embodiment provides the advantage that the cut does not run through the eye colorant. The ablative removal removes advantageously the offset cornea tissue and the eye colorant.

Further, it is conceivably that some eye colorant may remain arranged on the bottom side of the cornea flap. This eye colorant might be additionally removed ablatively via a laser by a respective ophthalmological device, in particular the ablative ophthalmological device.

In an embodiment, the ophthalmological system and / or the access cutting ophthalmological device comprise a patient interface, with a contact body, which contact body brings an outer surface of the cornea in an applanated form in a state where the patient interface is applied on the eye, and the electronic circuit is configured to control the respective scanner system to move the focal spot at locations on the applanated locations of the cornea comprising at least partially the eye colorant and / or to move the focal spot inside the cornea to generate the cut extending at least partially from the outer surface of the cornea into the cornea, thereby forming the cornea flap. The patient interface may be used for the ablative removal and / or for cutting of the flap. It is also conceivably that the patient interface remains for both procedures the same and only the ophthalmological device is switched. In particular, the contact body may compensate deviations in the outer shape of the cornea for forming the desired cut and for processing the desired area.

In an embodiment, the electronic circuit of the ophthalmological system or the electronic circuit of the access cutting ophthalmological device is configured to control the respective scanner system to move the focal spot along a surface of the cornea, which was previously at least partially covered by the eye colorant for leveling the surface of these locations. The removal of the eye colorant may leave a cavity in the cornea, having at least partially the shape of the removed area. It might be desired to process the cavity, in particular to level the cavity such that the cornea may heal advantageously after treatment and that the eyesight is as unaffected as possible.

According to a further aspect, a computer program product comprising a non-transitory computer-readable medium having stored thereon computer program code for controlling a processor of an ophthalmological system for ablative removal of eye colorant arranged in a cornea of an eye of a patient is specified. The ophthalmological system comprises preferably a position measurement system configured to determine an area in the cornea comprising the eye colorant, preferably using images of the cornea, a laser source configured to generate a pulsed laser beam, a focusing optics configured to make the pulsed laser beam converge onto a focal spot in the cornea, a scanner system configured to deflect the pulsed laser beam to target locations in the cornea. The computer program code is configured to control the processor such that the processor directs the focal spot of the scanner system to process the determined area comprising at least partially the eye colorant for ablative removal of the eye colorant arranged in the cornea of the eye.

The computer program code is further configured to control the processor such that the processor directs the ophthalmological system, in particular the ablative ophthalmological device and / or the flap cutting ophthalmological device as described above and hereinafter.

### BRIEF DESCRIPTION OF THE DRAWINGS

The herein described disclosure will be more fully understood from the detailed description given herein below and the accompanying drawings, which should not be considered limiting to the invention described in the appended claims. The drawings in which:
- **Figure 1**: shows a block diagram that schematically illustrates an ophthalmological device for surgical treatment of a cornea of an eye of a patient, in particular for ablative removal of eye colorant and / or for forming a cut in the cornea, according to a first embodiment;
- **Figure 2**: shows a plurality of ophthalmological devices, which form in combination with each other the ophthalmological system for ablative removal of eye colorant;
- **Figure 3**: shows an eye of a patient along the z-direction of the eye towards the eye according to a first embodiment;
- **Figure 4**: shows a cross section view of the eye as shown in Figure 3;
- **Figure 5**: shows a first detailed view of an eye of a patient according to another embodiment;
- **Figure 6**: shows a second detailed view of an eye of a patient according to another embodiment;
- **Figure 7**: shows a sequence of Figures from Figure 7a to Figure 7e, which shows the process for ablative removal of eye colorant according to a first embodiment;
- **Figure 8**: shows a sequence of Figures from Figure 8a to 8e, which shows the process for ablative removal of eye colorant according to a second embodiment;
- **Figure 9**: a cross section of an eye of a patient according, indicating in particular a processing path along which the focal spot of the pulsed laser beam is moved;
- **Figure 10**: a cross section of an eye of a patient including a patient interface according to a first embodiment, indicating in particular a processing path along which the focal spot of the pulsed laser beam is moved;
- **Figure 11**: a cross section of an eye of a patient including a patient interface according to further embodiment, indicating in particular a processing path along which the focal spot of the pulsed laser beam is moved.

### DESCRIPTION OF THE EMBODIMENTS

Reference will now be made in detail to certain embodiments, examples of which are illustrated in the accompanying drawings, in which some, but not all features are shown. Indeed, embodiments disclosed herein may be embodied in many different forms and should not be construed as limited to the embodiments set forth herein; rather, these embodiments are provided so that this disclosure will satisfy applicable legal requirements. Whenever possible, like reference numbers will be used to refer to like components or parts.

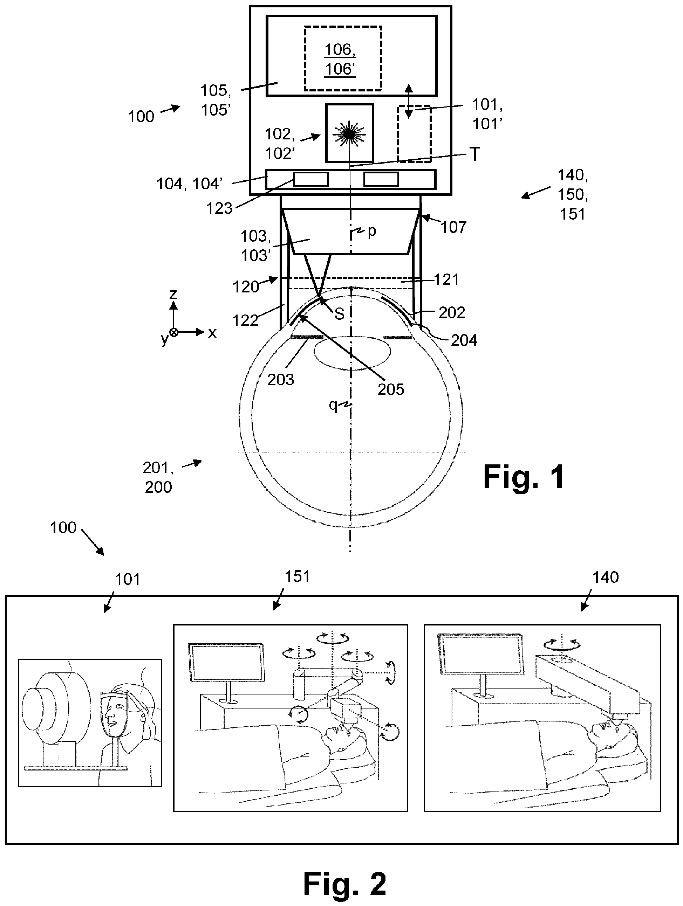

**Figure** 1 schematically shows an ophthalmological system 100 for surgical treatment of a cornea 202 of an eye 201 of a patient 200. The ophthalmological system 100 uses a pulsed laser beam T for treating the cornea 202. Specifically, the ophthalmological system 100 is configured to treat the cornea 202 and adjacently arranged eye tissue e.g. limbus sclera, lens etc., by irradiating or vaporizing the respective eye issue and / or material, e.g. eye colorant 204, arranged in the eye tissue, or by cutting the respective eye tissue. The person skilled in the art will understand that by controlling the ophthalmological system 100, in particular its components, respectively, it is possible to process three-dimensional (tissue) pieces in particular by ablatively removing eye tissue or material arranged in the cornea 202, e.g. predetermined areas 205, which may comprise eye colorant 204 as material in the eye 201. It is of course further possible to form three-dimensional cuts or pockets in the eye tissue. The person skilled in the art will further understand that by controlling the ophthalmological system 100, in particular its components, respectively, it might be further possible to achieve three-dimensional volume treatment of the eye tissue, e.g. for generating a pocket, a void volume inside the eye tissue e.g. the cornea 202.

As illustrated schematically in Figure 1, the ophthalmological system 100 comprises a laser source 102 for generating the pulsed laser beam T, a focusing optics 103 for focusing or making the pulsed laser beam T converge onto a focal spot S in or on the eye tissue, and a scanner system 104 for moving the focal spot S to target locations in and/or on the eye tissue, in particular configured to deflect the pulsed laser beam T to target locations in the cornea 202.

The ophthalmological system 100 further comprises an electronic circuit 105 for controlling the ophthalmological system 100, in particular the laser source 102 and the scanner system 104. The electronic circuit 105 implements a programmable control device and comprises e.g. one or more processors 106 with program and data memory and programmed software modules for controlling the processors 106, and/or other programmable circuits or logic units such as ASICs (application specific integrated circuits) or the likes. In an embodiment, a functional part of the electronic circuit 105 is arranged in a separate housing and implements a further programmable control device 105, e.g. a computer system, comprising e.g. one or more processors 106 with program and data memory and programmed software modules for controlling the processors 106, and/or other programmable circuits or logic units such as ASICs or the like. Depending on the embodiment and/or configuration, a communication link might be required, which comprises one or more electrical connections, an electronic bus, a wired communication network, and/or a wireless communication network.

The laser source 102 comprises e.g. a femtosecond laser for producing femtosecond laser pulses, which have pulse widths of typically 10 fs to 1000 fs (1 fs = 10 15 s). In another embodiment, the laser source 102 comprises a solid-state laser source or an excimer laser source. The laser source 102 is arranged in a separate housing or in a housing shared with the focusing optics 103.

The focusing optics 103 is configured to focus the pulsed laser beam T or the laser pulses, respectively, onto a focal spot S in or on the eye tissue, i.e. for making the pulsed laser beam T converge to a focus or focal spot in or on the eye tissue. The focusing optics or optical module 103 comprises one or more optical lenses. In an embodiment, the focusing optics 103 comprises a focus adjustment device for setting the focal depth of the focal spot S, for example one or more movable lenses, in the focusing optics 103 or upstream of the focusing optics 103, or a drive for moving the entire optics 103 along the projection axis p (z-axis). By way of example, the focusing optics 103 is installed in an application head 107, which can be placed onto the eye 201. The person skilled in the art will understand that in cases where the focusing optics 103 is adjusted (focus) or moved as part of the scanning process or scanning actuation, the focusing optics 103 and associated drives (actuators) can be viewed and considered as parts of the scanner system 104, implementing a z-scanner configured to modulate the depth z of the focal spot S along the projection axis p or the eye symmetry axis q.

As illustrated schematically in Figure 1, the ophthalmological system 100 comprises a patient interface 120 for attaching the application head 107 or the focusing optics 103, respectively, onto the eye 201. Depending on the embodiment, the patient interface 120 is connected to the application head 107 in a fixed or removable manner. The patient interface 120 comprises an optional contact body 121 and one or more suction elements configured to fix the contact body 121 and thus the patient interface 120 to the cornea 202. For example, the one or more suction elements are arranged in a fastening ring 122, e.g. a vacuum-controlled suction ring, whereby the one or more suction elements are connected fluidically to a suction pump. The contact body 121, also referred to as applanation body, is at least partly light-transparent. In the state where the patient interface 120 or the contact body 121, respectively, is fixed to the cornea 202, the cornea 202 is applanated where the contact body 121 is in contact with the exterior (anterior) surface of the cornea 202.

In an embodiment, the ophthalmological system 100 further comprises a position measurement system 101 configured to determine positional reference data of the cornea 202. Depending on the embodiment, the position measurement system 101 may comprise a video capturing system, an optical coherence tomography (OCT) system, and/or a structured light illumination system. Accordingly, the measurement data or positional reference data determined by the position measurement system 101 includes video data, including top view data (comprising two-dimensional images), and/or OCT data of the eye 201, in particular of the cornea 202 (comprising three-dimensional data, in particular three-dimensional tomography data). The position measurement system 101 is configured to determine the positional reference data of the cornea 202 also in an applanated state of the cornea 202. The position measurement system 101 is according to the embodiment shown in Figure 1 connected to and/or integrated with the electronic circuit 105, which is further configured to control ophthalmological system 100, in particular the scanner system 104, using the data, in particular the positional reference date or further data, from the position measurement system 101.

Aside from the optional positional reference data from the position measurement system 101, the electronic circuit 105 is configured to use treatment control data to control the ophthalmological device 100, in particular the scanner system 104 to move the focal spot S in or on the eye tissue to target locations along a processing path 160 defined by the treatment control data for treating in and/or on the eye tissue. The treatment control data is e.g. determined by the electronic circuit 105 using the position reference data from the position measurement system 101, stored in the data memory and/or data store of the electronic circuit 105 and/or received via a communication link from a separate computer system.

The position measurement system 101 is at least configured to determine an area 205 in the cornea 202, which comprises at least partially the eye colorant 202. The output of the position measurement system 101 is e.g. position reference data of the area 205 comprising eye colorant 202. The electronic circuit 105 is configured to determine treatment control data, in particular data for at least one processing path 160 along which the scanner system 104 moves the focal spot S or a scan line for treating the eye tissue, in particular for ablatively removing the eye colorant.

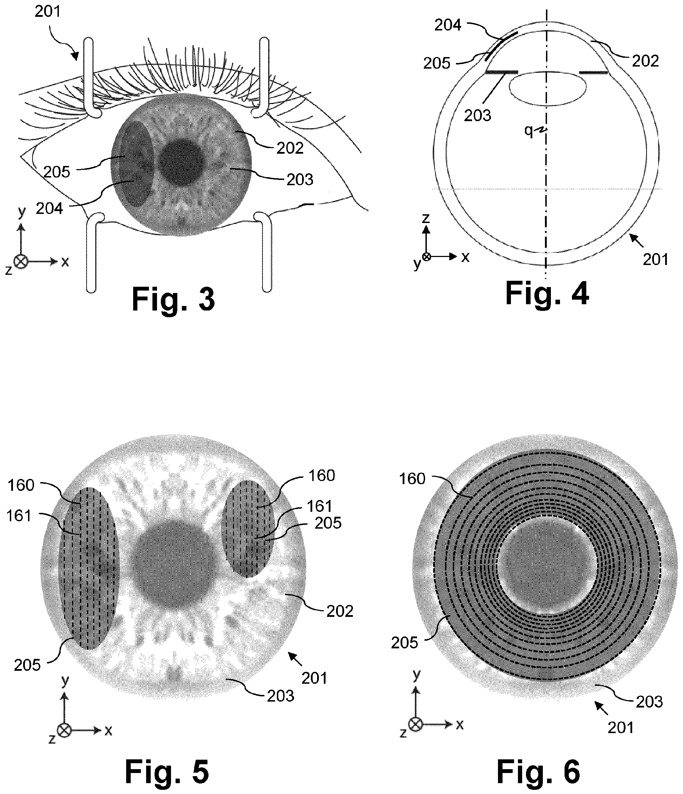

Further, the orientation, arrangement, geometrical parameters and physical parameters of the focal spot S or a scan line along the predetermined processing paths 160 might also be determined by the treatment control data. In effect, the treatment control data defines a sequence of consecutive target locations in a three-dimensional x/y/z-space for treating e.g. a three-dimensional area in the cornea 202. Depending on the embodiment and/or configuration, for defining the at least one processing path 160, the treatment control data comprises path definition data, e.g. coordinates, positions, positional references, etc., and/or path processing data, e.g. instructions, operations, and/or procedures for the scanner system 104 and its components, described below in more detail. As illustrated schematically in Figures 5 or 6, the at least one processing path 160 or the sub-processing paths 161 can be defined as a trajectory for the focal spot S or the scan line, to be moved along by the scanner system 104 for treating the cornea 202. The treatment control data may further comprise control data for controlling the laser source 102 e.g. for controlling physical parameters of the pulsed laser beam T like processing speed, pulse rate, pulse energy, etc..

Treating the eye tissue, in particular the cornea 202 imposes scanning requirements that must be met by a scanner system 104. More specifically, the scanning requirements are determined by the dynamics involved in moving the focal spot S and / or the scan line 130 along the processing path 160, defined by the treatment control data, for treating the eye tissue. The dynamics include the speed with which a focal spot is to be placed and moved along the scan line 130 and / or the processing path 160 and the distances that must be covered by the focal spot S within a given time, at a defined speed, with a defined acceleration, and/or with a defined speed of acceleration (jerk/jump). The scanning requirements include a depth-scanning requirement, representing the required modulation of the depth z of the focal spot S when moved along the processing path 160 defined in particular by the treatment control data. The depth-scanning requirement includes the dynamics required for the modulation of the depth z of the focal spot S, the scan line 130 respectively along the processing path 160 defined in particular by the treatment control data. The required depth scanning dynamics may comprise the required depth scanning speed, the required depth scanning frequency, the required amplitude of depth modulation at a particular speed of the depth modulation, the required amplitude of depth modulation at a particular frequency of the depth modulation, the required acceleration of the depth modulation, and/or the required speed of the acceleration of the depth modulation.

Treating of eye tissue, in particular of the cornea 202 may comprise to ablatively remove the eye colorant 204 and / or adjacent cornea tissue, and to form cuts 208 or pockets in the cornea 202. Treating is e.g. a collective term.

The ophthalmological system 100 as shown in Figure 1, with its components is e.g. used as ablative ophthalmological device 140 for ablative removal of eye colorant 204 arranged in the cornea. Further, the ophthalmological system 100 may additionally be used as access device 150, in particular as access cutting ophthalmological device 151. In addition, the position measurement system 101 is included in one device. In other words, the ophthalmological system 100 as shown in Figure 1 combines the ablative ophthalmological device 140, the position measurement system 101 and the access device 150 in one ophthalmological device. The access cutting ophthalmological device 151 may also comprise the position measurement system 101', the laser source 102', the focusing optics 103', the scanner system 104', the electronic circuit 105' etc. as indicated in Figure 1.

**Figure 2** shows another embodiment of an ophthalmological system 100. According to this embodiment, the ophthalmological system 100 comprises three separate devices, namely the position measurement system 101, the access cutting ophthalmological device 151 and the ablative ophthalmological device 140. The data from the positon measurement system 101 is advantageously transmitted to the access cutting ophthalmological device 151, such that the desired cornea flap 207 may be formed, and the data from the position measurement system 101 is additionally preferably transmitted to the ablative ophthalmological device 140 such that determined area 205 can be processed for ablative removal as desired. The patient 200 is e.g. measured by the position measurement system 101 and at a later point in time, e.g. after some days, the patient 200 is treated by the access cutting ophthalmological device 151, for cutting the cornea flap 207, the access cutting ophthalmological device 151 is moved away and the ablative ophthalmological device 140 is positioned respectively for the ablative treatment, in particular the ablative removal of eye colorant 205. In an embodiment, the patient interface 120 arranged on the eye 201 of the patient is not changed. The respective application head 107 of both devices is attached respectively. A combination of the embodiments is of course also conceivable.

**Figure 3** shows an eye 201 of a patient 200 along the z-axis when viewed towards the eye 201. The eye 201 comprises the iris 203 centrally arranged and the cornea 202 arranged above the iris 203 and the pupil of the eye 201. Figure 3 further indicates an area 205 of the cornea 203, which comprises the eye colorant 204. The area 205 has according to this embodiment an elliptical shape and was inserted e.g. for covering a defect in the iris 203 at the respective positon. The area 205 comprising eye colorant 204 is e.g. determined by the position measurement system 101.

**Figure 4** shows a cross section view of the eye 201 of the patient as shown in Figure 3. Figure 4 shows the cornea 202 of the patient 200, which comprises the area 205 comprising the eye colorant 204. Figure 4 shows advantageously that the eye colorant 204 is arranged partially above the iris 203 for covering partially the iris 203 when viewed along the z-axis towards the eye 201. In other embodiments, as shown e.g. in Figure 6, the entire iris 203 may be covered by eye colorant 204.

**Figure 5** shows a first detailed view of the eye 201 of a patient 200 according to another embodiment. According to this embodiment, the cornea 202 comprises two areas 205, which comprise eye colorant 205. The eye colorant 205 was e.g. inserted for covering the respective portions of the iris 203. Now, the eye colorant 205 may needs to be removed or replaced. Figure 5 additionally indicates a processing path 160, in particular two sub processing paths 161 one for each area 205 along which the focal spot S or a respective scan line is moved for processing, in particular for ablative removal of the eye colorant 204. Both sub-processing path 161 comprise a plurality of parallel lines along which the focal spot S is moved for ablative removal of the eye colorant 204.

**Figure 6** shows a second detailed view of the eye 201 of a patient 200 according to another embodiment. According to this embodiment, the area 205 comprising the eye colorant 204 has a ring shape or an annulus shape and covers almost the entire iris 203 of the eye. Figure 6 further indicates the processing path 160, which comprises a plurality of concentric arranged circles. A spiral shape or any other shape would of course also be conceivable. In another embodiment, the area 205 may be processed several times in order to process the area 205 throughout its entire thickness.

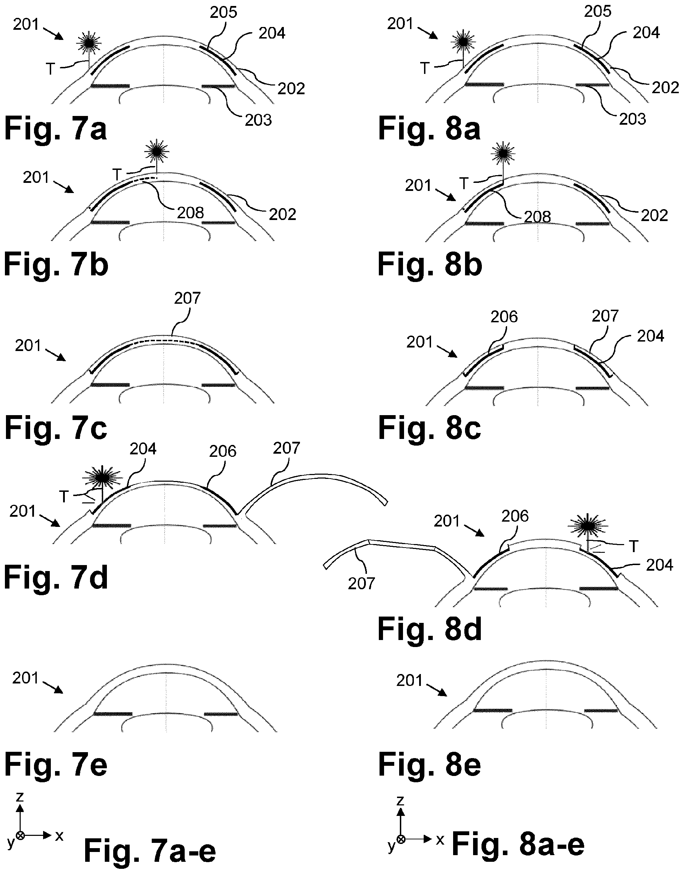

**Figure 7** shows a sequence of Figures from Figure 7a to Figure 7e, which shows the process for ablative removal of eye colorant 204 according to a first embodiment. The Figures 7a to 7 c show the process of forming or cutting of the cornea flap 207 using e.g. the access cutting ophthalmological device 151. Figure 7a shows the eye 201 comprising the eye colorant 204 in the respective area 205 for covering the iris 203. In addition, Figure 7a indicates a pulsed laser beam T from the access cutting ophthalmological device 151 for forming or cutting the cornea flap 207. Figure 7b shows the forming of the cornea flap 207, in particular, a cut through the cornea 202 partially along the upper surface of the eye colorant 204 is indicated by dashed lines. Figure 7c shows the eye 201 with the finished formed cornea flap 207. Figure 7d shows the eye 201 being treated by the ablative ophthalmological device 140. For enabling direct access, the cornea flap 207, which is according to this embodiment not completely separated from the rest of the eye tissue, is flipped away, which exposed the eye colorant 204, in particular the eye colorant comprising surface 206. The pulsed laser beam T of the ablative ophthalmological device 140 is moved along the processing path 160 for treating, in particular for ablatively removing the eye colorant 204. It is conceivable that some eye colorant 204 may remain on the underside of the cornea flap 207. This eye colorant may further be removed by a respective pulsed laser beam T prior of flipping or moving the cornea flap 207 back on the cornea 202. In case some eye colorant 204 remains on the cornea flap 207, the cornea flap 207 may be completely removed from the rest of the cornea 202 and treated at a different place with a respective puled laser beam from a respective ophthalmological device. Figure 7e shows the eye 201 after the ablative removal and after the cornea flap 207 has been flipped back. In another embodiment, new eye colorant 204 may be inserted, in particular into the cavity, which is left behind by the removed eye colorant 204 and removed cornea tissue, in particular prior of flipping the cornea flap 207 back.

**Figure 8** shows a sequence of Figures from Figure 8a to 8e, which shows the process for ablative removal of eye colorant 204 according to a second embodiment. This embodiment differs from the embodiment shown in Figure 7 at least in the shape of the cornea flap 207. The cornea flat 207 according to Figure 8 is formed by the access cutting ophthalmological device 151 and has an annulus or ring shape. The center area of the cornea 202 is spared by the pulsed laser beam T. In other words, the cornea flap 207 is only formed along the top surface 206 of the eye colorant 204 (including access cuts from the outer surface of the cornea 209). Figure 8b show the process of forming the respective annulus shaped cornea flap 207 and Figure 8c shows the finished cornea flap 207 still on the eye colorant 204. Figure 8d shows the annulus shaped cornea flap 207 flipped away exposing the eye colorant 204, in particular exposing the eye colorant comprising surface 206. Figure 8d further shows the pulsed laser beam T from the ablative ophthalmological device 140 moving along the processing path 160 for ablatively removing the eye colorant 204. Figure 8e shows the eye 201 after the ablative removal and after the cornea flap 207 has been flipped back.

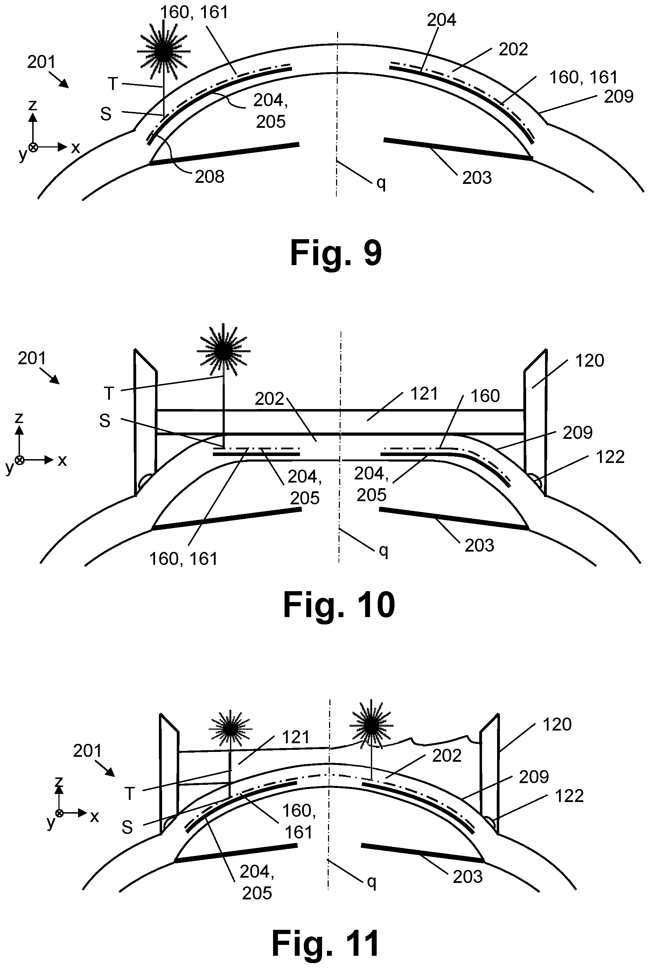

**Figure 9** shows a cross section of an eye 201 of a patient 200. The cross section indicates in particular the pulsed laser beam following the processing path 160 according to a first embodiment. Figure 9 shows in particular the iris 203, the cornea 202, the sclera the limbus of the eye 201 and the eye colorant 204 arranged in the cornea 202. Figure 9 shows that the processing paths 160 follow the contour of the cornea 202. In other words, the processing paths 160 have different depth positions or positions in z-direction from the peripheral portion of the cornea 202 towards the center area of the cornea 202. In particular, the depth of the focal spot S is controlled by the ophthalmological device 100, in particular by the focusing optics 103, such that the focal depth increases (moves away in z-direction from the eye center) when the focal spot S is moved towards the center area of the cornea 202. It is thereby possible to move the focal spot S in three dimensions within the cornea 202, such that the cut 208 for forming the cornea flap 207 can advantageously follow the three dimensional shape, in particular the thickness, of the area 205 and / or such that the area 205 comprising the eye colorant 204 can advantageously be processed for ablatively removing the eye colorant 204. As indicated in Figure 9, removing of the eye colorant 204 may be performed without forming and flipping the cornea flap 207 away. Figure 9 schematically indicates the pulsed laser beam T of the ablative ophthalmological device 140 and / or of the access cutting ophthalmological device 151.

**Figure 10** shows a cross section of an eye 201 of a patient 200. The cross section indicates in particular the pulsed laser beam T and the respective focal spot S following at least one of the predetermined processing paths 160 according to a further embodiment. According to this embodiment, a patient interface 120 is arranged on the eye 201 of the patient. The patient interface 120 comprises a contact body 121 which contacts and ap-planates the cornea 202, in particular the outer surface 209 of the cornea 202. The patient interface 120 further comprises the fastening ring 122, which is configured to position the patient interface 120 on the eye 201. The fastening ring 122 might comprise the suction opening, which provides a vacuum for advantageous fixation. Figure 10 further shows two deviating areas 205 comprising eye colorant 204, which results also in different sub-processing paths 161 along which the focal spot might be moved for forming the cut 208 and / or for ablative removal. According to the first embodiment, shown left, the processing path 160 is arranged straight, with respect to the z-direction, the focal depth is constant. Further, the area 205 and therefore the sub-processing path 161 according to the embodiment shown left is completely arranged in the applanating zone of the cornea 202. According to the embodiment shown right, the sub-processing path 161 additionally extends beyond the applanating zone and follows the lateral shape of the area 205 comprising the eye colorant 204. In other words, the sub-processing path 161 might be divided into a first straight portion and a second portion with varying focal depth. The patient interface 120 is e.g. configured to be connected to the ablative ophthalmological device 140 and to the access cutting ophthalmological device in case both devices are separate devices forming in combination the ophthalmological system 100. In this case, the patient interface 120 may remain on the eye 210 when the devices are switched.

**Figure 11** shows a cross section of an eye 201 of a patient 200. The cross section indicates in particular the pulsed laser beam T and the respective focal spot S following at least one of the predetermined processing paths 160 according to two additional embodiment. The embodiment as shown left and the embodiment as shown right. The embodiment shown left deviates from the embodiment shown in Figure 10 at least in the contact body 121 of the patient interface 120. The contact body 121 of the embodiment shown left follows the natural shape of the cornea 202. In other words, the outer surface of the contact body 121, which is configured to contact he cornea 202 has e.g. a spherical shape, such that contact body 121 adapts advantageously to the outer surface 209 of the cornea 202. The embodiment shown right deviates from the previously mentioned embodiment in that the patient interface 120 does not comprise a contact body. According to this embodiment, a liquid solution is arranged in the patient interface 120 in particular between the application head 107 and the outer surface 209 of the cornea 202. This embodiment of the patient interface might be called liquid interface.

### LIST OF REFERENCE SYMBOLS

- 100: ophthalmological system
- 101, 101': position measurement system
- 102, 102': laser source
- 103, 103': focusing optics
- 104, 104': scanner system
- 105, 105': electronic circuit
- 106: processor
- 107: application head
- 108: arm
- 120: patient interface
- 121: contact body
- 122: fastening ring
- 123: scanner device
- 140: ablative ophthalmological device
- 150: access device
- 151: access cutting ophthalmological device
- 160: processing path
- 161: sub processing paths
- 200: patient
- 201: eye
- 202: cornea
- 203: iris
- 204: eye colorant
- 205: area comprising the eye colorant
- 206: eye colorant comprising surface
- 207: cornea flap
- 208: cut
- 209: outer surface of the cornea
- S: Focal spot
- T: Pulsed laser beam
- p: projection axis
- q: symmetry axis

## Claims

1. An ophthalmological system (100) for ablative removal of eye colorant (204) arranged in a cornea (202) of an eye (201) of a patient (200), the ophthalmological system (100) comprising
a. position measurement system (101) configured to determine an area (205) in the cornea (202) comprising the eye colorant (204);
b. a laser source (102) configured to generate a pulsed laser beam (T);
c. a focusing optics (103) configured to make the pulsed laser beam (T) converge onto a focal spot (S) in the cornea (202);
d. a scanner system (104) configured to deflect the pulsed laser beam (T) to target locations in the cornea (202); and
e. an electronic circuit (105) configured to control the scanner system (104) to direct the focal spot (S) to process the determined area (205) comprising the eye colorant (204) in the cornea (202) for ablative removal of the eye colorant (204) arranged in the cornea (202).

2. The ophthalmological system (100) according claim 1, wherein the position measurement system (101) is configured to determine at least one of: position reference data of the cornea (202), position reference data of the area (205) comprising the eye colorant (204) to be treated or position reference data of the treated area (205), during processing or treating of the cornea (202), in particular continuously, such that treating, in particular the ablative removal of the eye colorant (202), is monitorable, in particular continuously monitorable.

3. The ophthalmological system (100) according to any one of the preceding claims, wherein the laser source (102) of the ophthalmological system (100) used for ablative removal of the eye colorant (204) comprises at least one of: a solid-state laser source, excimer laser source or a femtosecond laser source.

4. The ophthalmological system (100) according to any one of the preceding claims, wherein the electronic circuit (105) is configured to control a scanner device (123) of the scanner system (104) to move the focal sport (S) with a scanning speed along a processing path (160) generated based on the determined area (205) comprising the eye colorant (204).

5. The ophthalmological system (100) according to any one of the preceding claims, wherein the position measurement system (101) is configured to determine at least one of: a thickness of the area (205) comprising the eye colorant (202) within the cornea (202) or a depth position of the area (205) comprising the eye colorant (202) within the cornea (202), and wherein the electronic circuit (105) is configured to move the focal spot (S) to process the determined area (205) comprising the eye colorant (202), using the determined thickness and / or depth position of the area (205) comprising the eye colorant (202) and/or wherein the electronic circuit (105) is further configured to control at least one laser parameter of the puled laser beam (T) to process the determined area (205) using the determined thickness and / or depth position of the area (205).

6. The ophthalmological system (100) according to any one of the preceding claims, wherein the electronic circuit (105) is further configured to control the scanner system (104) to move the focal spot (S) to form a cut (208) extending at least partially from the outer surface (209) of the cornea (202) into the cornea (202), thereby forming a cornea flap (207), which is moveable for providing direct access to the area (205) comprising the eye colorant (204) in the cornea (202).

7. The ophthalmological system (100) according to any one of the claims 1 to 5, wherein the ophthalmological system (100) further comprising:
a. an access device (150) configured to form a cut (208) in the cornea (202), which extends from an outer surface of the cornea (202) into the cornea (202) thereby forming a cornea flap (207), which is moveable for providing direct access to the area (205) comprising the eye colorant (204) in the cornea (202).

8. The ophthalmological system (100) according to claim 7, wherein the access device (150) is provided by a standalone access cutting ophthalmological device (151) comprising
a. a position measurement system (101') configured to determine an area (205) in the cornea (202) comprising the eye colorant (204);
b. a laser source (102') configured to generate a pulsed laser beam (T');
c. a focusing optics (103') configured to make the pulsed laser beam (T') converge onto a focal spot (S') in the cornea (202);
d. a scanner system (104') configured to deflect the pulsed laser beam (T') to target locations in the cornea (202); and
e. an electronic circuit (105') configured to control the scanner system (104') to move the focal spot (S') inside the cornea (202) to form the cut (208) extending at least partially from the outer surface (209) of the cornea (202) into the cornea (202) using the determined area (205), thereby forming the cornea flap (207), which is moveable for providing direct access to the area (205) comprising the eye colorant (202).

9. The ophthalmological system (100) according to claim 8, wherein the laser source (102') of the access cutting ophthalmological device (151) comprises at least one of: a femtosecond laser source for producing femtosecond laser pulses, a solid-state laser source or an excimer laser source.

10. The ophthalmological system (100) according to one of the claims 8 or 9, wherein the electronic circuit (105') is configured to control the scanner system (104') to move the focal spot (S) inside the cornea (202) to generate the cut (208) such that a portion of the cornea flap (207) remains in contact with the rest of the cornea (202), enabling that the cornea flap (207) is flipable away for enabling access and flipable back for enabling to cover the area (205), which previously comprised the eye colorant (204).

11. The ophthalmological system (100) according to one of the claims 8 to 10, wherein the electronic circuit (105') is configured to control the scanner system (104') to move the focal spot (S) inside the cornea (202) to generate an at least partially annulus shaped cut (208), thereby forming an at least partially annulus shaped cornea flap (207), which is moveable for providing direct access to the eye colorant comprising location (205), and which keeps the center portion of the cornea (202) unaffected.

12. The ophthalmological system (100) according to one of the claims 8 to 11, wherein the electronic circuit (105') is configured to control the scanner system (104') to move the focal spot (S) inside the cornea (202) to generate the cut (208) such the cut (208) is arranged adjacent to the area (205) comprising the eye colorant (204).

13. The ophthalmological system (100) according to one of the preceding claims, wherein the ophthalmological system (100) and / or the access cutting ophthalmological device (151) comprise a patient interface (120), with a contact body (121), which contact body (121) brings an outer surface (209) of the cornea (202) in an applanated form in a state where the patient interface (120) is applied on the eye (201); and the electronic circuit (105, 105') is configured to control the respective scanner system (104, 104') to move the focal spot (S) at locations on the applanated locations of the cornea (202) comprising at least partially the eye colorant (104) and / or to move the focal spot (S) inside the cornea (202) to generate the cut (208) extending at least partially from the outer surface (209) of the cornea (202) into the cornea (202), thereby forming the cornea flap (207).

14. The ophthalmological system (100) according to one of the one of the preceding claims, wherein the electronic circuit (105) of the ophthalmological system (100) or the electronic circuit (105') of the access cutting ophthalmological device (151) is configured to control the respective scanner system (104, 104') to move the focal spot (S) along a surface of the cornea (202), which was previously at least partially covered by the eye colorant (204) for leveling the surface of these locations.

15. A computer program product comprising a non-transitory computer-readable medium having stored thereon computer program code for controlling a processor (106) of an ophthalmological system (100) for ablative removal of eye colorant (204) arranged in a cornea (202) of an eye (201) of a patient (200), which comprises position measurement system (101) configured to determine an area (205) in the cornea (202) comprising the eye colorant (202), preferably using images of the cornea (202), a laser source (102) configured to generate a pulsed laser beam (T), a focusing optics (103) configured to make the pulsed laser beam (T) converge onto a focal spot (S) in the cornea (202), a scanner system (104) configured to deflect the pulsed laser beam (T) to target locations in the cornea (202); and, whereby the computer program code is configured to control the processor (106) such that the processor (106): directs the focal spot (S) of the scanner system (104) to process the determined area (205) comprising at least partially the eye colorant (204) for ablative removal of the eye colorant (204) arranged in the cornea (202) of the eye (201).
